Europäisches Patentamt

European Patent Office

Office européen des brevets

(19)

(11) Numéro de publication : **0 336 794 B1**

## (12) FASCICULE DE BREVET EUROPEEN

(45) Date de publication du fascicule du brevet :
**29.07.92 Bulletin 92/31**

(51) Int. Cl.$^5$ : **G01N 1/14**

(21) Numéro de dépôt : **89400671.7**

(22) Date de dépôt : **10.03.89**

(54) **Appareil pour l'analyse et la prise d'échantillons sur des effluents liquides issus d'une installation industrielle.**

(30) Priorité : **14.03.88 FR 8803286**

(43) Date de publication de la demande :
**11.10.89 Bulletin 89/41**

(45) Mention de la délivrance du brevet :
**29.07.92 Bulletin 92/31**

(84) Etats contractants désignés :
**DE ES GB IT NL**

(56) Documents cités :
**DE-A- 2 641 801**
**FR-A- 2 264 278**
**FR-A- 2 570 824**
**US-A- 3 015 957**
**US-A- 4 151 086**
**PATENT ABSTRACTS OF JAPAN, vol. 3, no. 48**
**(E-106), 24th April 1979 & JP A 5425789**

(73) Titulaire : **AEROSPATIALE Société Nationale Industrielle**
**37, Boulevard de Montmorency**
**F-75781 Paris Cédex 16 (FR)**

(72) Inventeur : **Jonca, Henri Valentin Jean**
**30, Boulevard Jean Brunhes**
**F-31300 Toulouse (FR)**

(74) Mandataire : **Mongrédien, André et al**
**c/o SOCIETE DE PROTECTION DES INVENTIONS 25, rue de Ponthieu**
**F-75008 Paris (FR)**

EP 0 336 794 B1

Jouve, 18, rue Saint-Denis, 75001 PARIS

## Description

L'invention concerne un appareil conçu pour effectuer automatiquement et à intervalles réguliers des analyses et des prélèvements d'échantillons sur les effluents liquides rejetés par une installation industrielle.

La plupart des installations industrielles rejettent des effluents qui sont soumis à une surveillance toujours plus accrue de la part des services publics. En particulier, dans le cas des ateliers de traitements chimiques industriels et notamment de traitements de surfaces, la quantité de chacun des métaux rejetés dans les effluents liquides doit pouvoir être mesurée de façon de plus en plus précise.

Actuellement, cette surveillance est effectuée le plus souvent manuellement par un ou deux prélèvements journaliers accompagnés d'une mesure du débit des effluents dans la canalisation de rejet de l'installation. On analyse ensuite chaque échantillon pour déterminer la concentration de certains éléments chimiques, et notamment des métaux, dans cet échantillon. La quantité de chacun des éléments chimiques rejetés chaque jour est ensuite calculée en tenant compte du débit mesuré par ailleurs.

Cette technique de surveillance est évidemment très imprécise puisqu'elle ne tient pas compte de l'évolution de la composition des rejets en cours de journée, qui peut parfois être très importante. De plus, elle présente un temps de réaction relativement long qui peut parfois avoir des conséquences graves sur l'environnement.

Les techniques de surveillance existantes ne sont donc plus adaptées aux exigences de précision et de rapidité imposées par les pouvoirs publics.

Un appareil conforme au préambule de la revendication 1 est connu du document FR-A-2 570 824.

Par ailleurs, il est connu du document US-A-4 151 086 de prévoir un ensemble d'analyse dans un appareil analogue, placé dans une canalisation de rejet équipée d'une vanne.

Enfin, il est connu du document FR-A-2 264 278 de prévoir un opacimètre dans un appareil de surveillance des rejets.

L'invention a précisément pour objet un appareil permettant d'analyser automatiquement et à intervalles réguliers les effluents liquides rejetés par une installation industrielle, afin de connaître avec précision la quantité de certains éléments chimiques tels que des métaux rejetés et de pouvoir réagir de façon pratiquement immédiate si un seuil critique est atteint.

L'invention a aussi pour objet ion appareil permettant de prélever un échantillon représentatif de chacune des analyses effectuées, afin de permettre un contrôle des résultats de ces analyses lorsqu'un doute existe sur ces résultats.

L'invention a aussi pour objet un appareil permettant de mesurer en continu l'opacité des effluents liquides rejetés par l'installation, afin de permettre une réaction immédiate dans le cas où une coloration brutale de l'eau se produirait entre deux analyses effectuées par l'appareil.

Conformément à l'invention, ces différents objectifs sont atteints grâce à un appareil pour l'analyse et la prise d'échantillons sur des effluents liquides sortant d'une installation industrielle par une canalisation de rejet, comprenant :
- un premier tube de prélèvement raccordé sur la canalisation de rejet, équipé d'un premier organe de pompage et alimentant un ensemble de prise d'échantillons ;
- un moyen de mesure de débit dans la canalisation de rejet ; et
- un ensemble de commande actionnant automatiquement le premier organe de pompage, l'ensemble de prise d'échantillons et le moyen de mesure de débit en fonction desdits paramètres et recevant des signaux délivrés par le moyen de mesure de débit pour déterminer lesdites informations ;

caractérisé par le fait que, la canalisation de rejet étant équipée d'une vanne :
- le premier tube de prélèvement alimente simultanément un ensemble d'analyse mesurant la concentration d'au moins un élément chimique dans les effluents; cet ensemble d'analyse comprenant une première pipette de prélèvement interposée entre le premier tube de prélèvement et un tube d'analyse, placé à un niveau inférieur à celui de la pipette, cette dernière comportant une vanne supérieure et une vanne inférieure actionnées par l'ensemble de commande, un tube de vidange, également équipé d'une vanne actionnée par l'ensemble de commande, reliant le fond du tube d'analyse à la canalisation de rejet, l'ensemble d'analyse comprenant aussi des réservoirs contenant différents réactifs et situés à un niveau supérieur à celui du tube d'analyse, chaque réservoir étant relié au tube d'analyse par un tube équipé d'une vanne actionnée par l'ensemble de commande ;
- un deuxième tube de prélèvement, raccordé sur la canalisation de rejet et équipé d'un deuxième organe de pompage alimente un opacimètre ; et
- l'ensemble de commande est équipé d'un organe d'entrée de paramètres et d'un organe de sortie d'informations, cet ensemble de commande actionnant aussi automatiquement le deuxième organe de pompage, l'ensemble d'analyse et l'opacimètre, et recevant aussi des signaux délivrés par l'ensemble d'analyse et par l'opacimètre.

Un tel appareil, qui est monté à demeure sur la canalisation de rejet de l'installation, permet d'effectuer périodiquement une analyse et un prélèvement d'échantillons, de mesurer le débit des effluents au moment où chaque analyse est effectuée, et de contrôler en continu l'opacité de ces effluents.

Bien que les informations affichées par l'organe de sortie de l'ensemble de commande puissent être exploitées manuellement par un opérateur, l'appareil est équipé avantageusement d'au moins une alarme qui est actionnée automatiquement par l'ensemble de commande lorsque l'un au moins des signaux franchit un seuil constituant l'un desdits paramètres.

De façon comparable, l'ensemble de commande assure avantageusement la fermeture automatique de la vanne équipant la canalisation de rejet lorsque l'un au moins des signaux franchit un seuil constituant l'un desdits paramètres.

Dans un mode de réalisation préféré de l'invention, l'ensemble de prise d'échantillons comprend une deuxième pipette de prélèvement reliée au premier tube de prélèvement et placée de façon à se trouver en permanence au-dessus d'un flacon porté par un dispositif d'avance pas à pas, cette deuxième pipette comportant une vanne supérieure et une vanne inférieure actionnées par l'ensemble de commande, un tube de rinçage également équipé d'une vanne actionnée par l'ensemble de commande reliant le fond de la pipette à la canalisation de rejet.

Afin de compléter la surveillance, un moyen de mesure de Ph relié à l'ensemble de commande est placé de préférence dans la pipette de prélèvement.

Dans le mode de réalisation préféré de l'invention, l'ensemble d'analyse comprend un dispositif d'analyse tel qu'un photomètre muni d'une source lumineuse et d'un sélecteur de filtres entraîné par un moteur pas à pas actionné par l'ensemble de commande. Pour chacun des réactifs, un filtre approprié peut ainsi être sélectionné. Selon le mode de réalisation préféré de l'invention, l'opacimètre comprend un tube transparent dans lequel circulent les effluents, au moins une source lumineuse placée latéralement le long du tube d'analyse, des cellules photoélectriques placées en vis-à-vis de la source lumineuse, de l'autre côté du tube d'analyse, et un moyen pour calculer la somme des signaux délivrés par les cellules, ce moyen étant actionné par l'ensemble de commande, qui reçoit en retour un signal représentatif de cette somme.

Un exemple de réalisation de l'invention va maintenant être décrit, à titre d'exemple nullement limitatif, en se référant aux dessins annexés dans lesquels :

– la figure 1 est une vue schématique représentant sous la forme d'un schéma bloc l'appareil d'analyse et de prise d'échantillons selon l'invention ;

– la figure 2 représente une partie de l'appareil de la figure 1 et notamment l'ensemble de prise d'échantillons et l'opacimètre ; et

– la figure 3 représente une autre partie de l'appareil de la figure 1, comportant notamment l'ensemble d'analyse servant à mesurer la concentration de certains éléments chimiques dans les effluents.

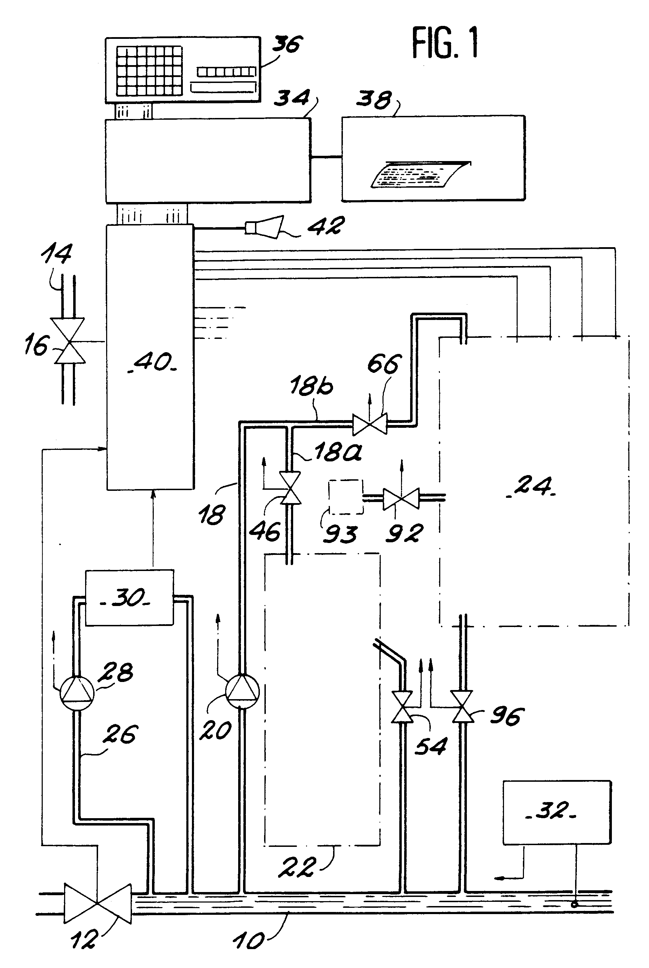

Sur la figure 1, la référence 10 désigne une canalisation de rejet équipant une installation industrielle telle qu'un atelier de traitement de surfaces. Cette canalisation 10 est équipée d'au moins une vanne 12 permettant de stopper le rejet des effluents liquides si cela s'avère nécessaire.

L'installation est également équipée d'au moins une canalisation d'arrivée d'eau 14 munie d'une vanne 16 permettant d'interrompre l'arrivée d'eau en cas de besoin.

Conformément à l'invention, l'installation industrielle est également équipée d'un appareil permettant d'effectuer à intervalles réguliers des analyses et des prises d'échantillons sur les effluents rejetés par la canalisation 10 et de contrôler en continu l'opacité de ces effluents, toutes ces opérations étant effectuées de façon automatique.

A cet effet, l'appareil selon l'invention comprend un premier tube de prélèvement 18 raccordé sur la canalisation de rejet 10 et équipé d'une pompe 20. En aval de la pompe 20, le tube de prélèvement 18 se divise en deux branches 18a et 18b qui alimentent respectivement un ensemble de prise d'échantillons 22 et un ensemble d'analyse 24.

L'appareil selon l'invention comporte également un deuxième tube de prélèvement 26 raccordé en dérivation sur la canalisation de rejet 10 et équipé d'une pompe 28. Ce tube de prélèvement 26 permet de faire passer en continu une partie des effluents liquides rejetés par la canalisation 10 dans un opacimètre 30.

Pour compléter les données nécessaires à la surveillance des effluents rejetés par l'installation, l'appareil selon l'invention comprend aussi un débitmètre 32 mesurant le débit des effluents liquides dans la canalisation de rejet 10.

Afin de commander son fonctionnement de façon automatique et de permettre un traitement et une exploitation immédiate des signaux délivrés par l'ensemble d'analyse 24, par l'opacimètre 30 et par le débitmètre 32, l'appareil selon l'invention comprend également un ensemble de commande constitué dans l'exemple de réalisation représenté par un microprocesseur 34. Ce microprocesseur 34 est équipé d'un clavier de commande 36 permettant, avant la mise en oeuvre de l'appareil, d'introduire certains paramètres nécessaires à son fonctionnement. Le microprocesseur 34 est également équipé d'un organe de sortie tel qu'une imprimante 38 permettant d'afficher les informations nécessaires à la surveillance des rejets. L'imprimante 38 peut le cas échéant être remplacée ou assistée par un autre système d'affichage tel qu'un écran.

D'une manière en elle-même classique, le microprocesseur 34 est relié aux différentes parties de l'appareil qui doivent être pilotées et qui lui fournissent les signaux nécessaires à l'élaboration des informations affichées sur l'imprimante 38 au travers d'une interface 40. Dans l'exemple de réalisation représenté sur la figure 1, l'interface 40 est également reliée à une ou plusieurs alarmes sonores et/ou visuelles 42 ainsi qu'aux vannes 12 et 16 placées respectivement dans la canalisation de rejet 10 et dans la canalisation d'entrée d'eau 14.

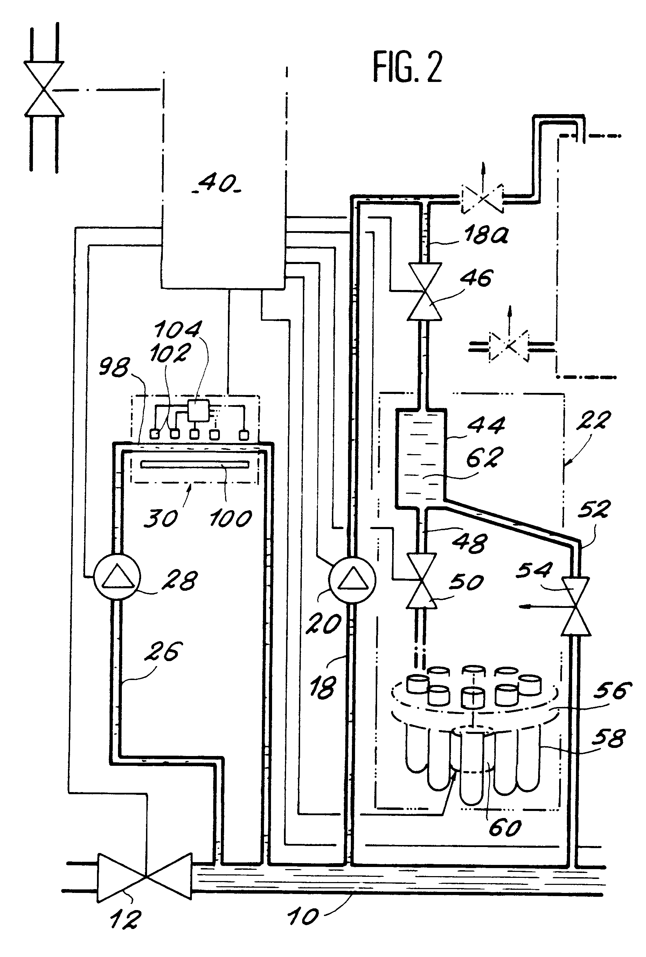

L'ensemble de prise d'échantillons 22 va maintenant être décrit plus en détail en se référant à la figure 2.

Comme l'illustre cette dernière figure, la branche 18a du tube de prélèvement 18 débouche à l'extrémité supérieure d'une première pipette de prélèvement 44 au travers d'une vanne supérieure 46. A son extrémité inférieure, la pipette 44 est munie d'un tube verseur vertical 48 muni d'une vanne inférieure 50. Le volume de la pipette 44 entre les vannes 46 et 50 détermine le volume de chaque échantillon d'effuents prélevé dans la canalisation de rejet 10. Un tube de rinçage 52, également équipé d'une vanne 54, relie par ailleurs le fond de la pipette 44 à la canalisation de rejet 10.

L'ensemble de prise d'échantillons 22 comprend de plus un plateau ou panier tournant 56, d'axe vertical, supportant un certain nombre de flacons ou de récipients analogues 58, conçus pour recevoir chacun l'un des échantillons prélevés. Les flacons 58 sont donc initialement vides et leur nombre correspond au nombre d'échantillons que l'on désire prélever au cours d'une période d'utilisation de l'appareil. A titre d'exemple, le plateau 56 peut supporter quarante flacons 58 si l'appareil est conçu pour fonctionner pendant dix heures et pour effectuer un prélèvement environ toutes les quinze minutes. L'agencement du plateau 56 est tel qu'un flacon 58 se trouve en permanence en dessous de l'extrémité inférieure du tube 48. Le plateau 56 est entraîné en rotation autour de son axe par un moteur pas à pas 60 dont l'actionnement permet d'amener un nouveau flacon vide 58 sous le tube 48 lorsqu'un prélèvement a été effectué.

Dans l'ensemble de prise d'échantillons 22 qui vient d'être décrit, chacune des vannes 46, 50 et 54 ainsi que le moteur pas à pas 60 sont actionnés par le microprocesseur 34 au travers de l'interface 40. Le microprocesseur 34 commande également la pompe 20, dont l'actionnement à intervalles réguliers permet d'effectuer simultanément le prélèvement d'un échantillon à l'aide de l'ensemble 22 et une analyse à l'aide de l'ensemble 24, comme on le verra ultérieurement.

Pour compléter la surveillance des effluents liquides rejetés par la canalisation 10, l'appareil selon l'invention comprend également, de préférence, un Ph-mètre 62 placé dans la pipette 44. Ce Ph-mètre est relié au microprocesseur 34 au travers de l'interface 40, de telle sorte que le microprocesseur assure la commande du Ph-mètre et reçoive les signaux délivrés par ce dernier pour en assurer le traitement et l'analyse et permettre une action immédiate si nécessaire.

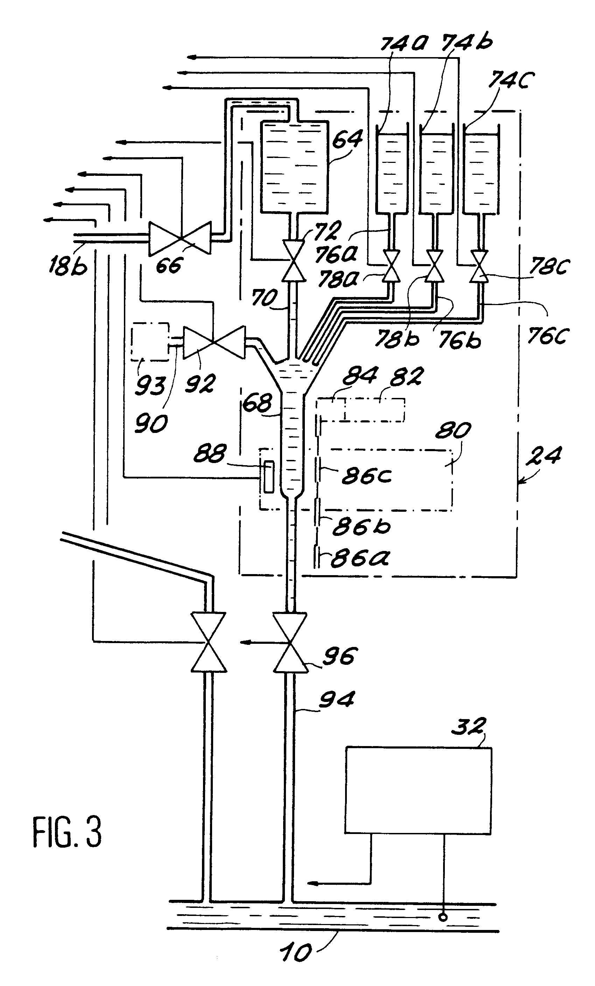

L'ensemble d'analyse 24 va maintenant être décrit plus en détail en se référant à la figure 3.

Comme l'illustre cette dernière figure, la branche 18b du tube de prélèvement 18 débouche à l'extrémité supérieure d'une deuxième pipette de prélèvement 64 et comporte, en amont de cette pipette, une vanne supérieure 66. La pipette 64 est placée à un niveau supérieur à celui d'un tube d'analyse 68 et reliée à ce dernier par un tube vertical 70 débouchant dans le fond de la pipette 64 et à la partie supérieure du tube d'analyse 68. Le tube 70 est équipé d'une vanne inférieure 72. Le volume délimité entre les vannes 66 et 72 à l'intérieur de la pipette 64 est choisi de façon à permettre un nombre déterminé d'analyses successives sur un même prélèvement, chacune de ces analyses ayant pour objet de mesurer la concentration d'un élément chimique déterminé, notamment d'un métal, dans l'échantillon prélevé. Ainsi, dans l'hypothèse où la concentration de huit éléments chimiques différents doit être mesurée successivement dans chacun des échantillons prélevés, le volume de la pipette 64 entre les vannes 66 et 72 est égal à huit fois le volume nécessaire à chacune des analyses.

L'ensemble d'analyse 24 comprend de plus un certain nombre de réservoirs 74a, 74b, 74c qui sont disposés, comme la pipette 64, à un niveau supérieur à celui du tube d'analyse 68. Le fond de chacun des réservoirs 74a, 74b, 74c est relié à la partie supérieure du tube d'analyse 68 par un tube d'alimentation 76a, 76b, 76c respectivement. Chacun des tubes d'alimentation 76a, 76b, 76c est équipé d'une vanne 78a, 78b, 78c respectivement.

Les réservoirs 74a, 74b, 74c contiennent chacun un réactif approprié à la mesure de la concentration de l'un des éléments chimiques que l'on désire surveiller dans les effluents rejetés par la canalisation 10. Par conséquent, le nombre de réservoirs correspond au nombre d'analyses à effectuer sur chacun des échantillons prélevés. Il existe par exemple huit réservoirs dans le cas où la concentration de huit éléments chimiques différents doit être surveillée dans les effluents.

La mesure de la concentration de chacun des éléments chimiques à surveiller est effectuée dans le tube d'analyse 68 au moyen d'un dispositif d'analyse 80 constitué dans l'exemple de réalisation représenté par un photomètre. Dans un autre mode de réalisation non représenté, le photomètre 80 peut être remplacé par un spectromètre de masse.

Lorsqu'un volume donné de l'échantillon contenu dans la pipette 64 et un volume donné de réactif contenu dans l'un des réservoirs 74a, 74b, 74c ont été introduits dans le tube d'analyse 68, un moteur pas à pas 82 commandant un sélecteur de filtres 84 permet d'associer au photomètre 80 l'un des filtres d'une série de filtres 86a, 86b, 86c. Comme les réactifs contenus dans les réservoirs 74a, 74b, 74c, chacun des filtres 86a, 86b, 86c est adapté à la mesure de la concentration de l'un des éléments chimiques à surveiller dans les effluents rejetés par la canalisation 10. D'une manière en elle-même connue, le photomètre 80 est équipé d'une source lumineuse 88 située de l'autre côté de l'échantillon contenu dans le tube d'analyse 68, par rapport à la partie détectrice et de mesure du photomètre 80.

Pour compléter la description de l'ensemble d'analyse 24, on voit sur la figure 3 qu'un tube de rinçage 90 équipé d'une vanne 92 débouche également dans la partie supérieure du tube d'analyse 68. A son extrémité opposée, le tube de rinçage 90 est relié à une source d'eau de rinçage 93 constituée par exemple par de l'eau déminéralisée. Le fond du tube d'analyse 68 est raccordé sur la canalisation de rejet 10 par un tube de vidange 94 muni d'une vanne 96.

La commande de chacune des vannes 66, 72, 92, 96, 78a, 78b, 78c est assurée par le microprocesseur 34 au travers de l'interface 40. Le microprocesseur 34 pilote également au travers de l'interface le moteur pas à pas 82 ainsi que le photomètre 80 et elle reçoit les signaux délivrés par ce dernier pour en effectuer le traitement et l'analyse, assurer l'affichage des résultats sur l'imprimante 38 et commander le cas échéant la mise en oeuvre des alarmes 42 et la fermeture des vannes 12 et 16.

En se référant à nouveau à la figure 2, on décrira maintenant plus en détail l'opacimètre 30.

L'opacimètre 30 comprend tout d'abord un tube transparent 98, par exemple en verre et d'une longueur d'environ 40 cm, dont chacune des extrémités est reliée à la canalisation de rejet 10 par le tube de prélèvement 26.

L'opacimètre 30 comprend également une source lumineuse 100 telle qu'un tube au néon disposé parallèlement au tube transparent 98, de façon à être situé latéralement le long de ce tube. Le tube au néon 100 peut être remplacé par plusieurs sources lumineuses ponctuelles disposées latéralement le long du tube 98. Des cellules photoélectriques 102, à sensibilité réglable, par exemple au nombre de huit, sont également disposées latéralement par rapport au tube transparent 98, du côté opposé au tube au néon 100, et régulièrement espacées le long du tube 98. Ainsi, la lumière émise par le tube 100 traverse les effluents circulant dans le tube 98 avant de parvenir aux cellules 102. L'intensité de la lumière reçue par chacune des cellules photoélectriques est donc fonction de l'opacité de l'effluent liquide circulant dans le tube transparent 98.

Un circuit sommateur 104 est de préférence associé à l'ensemble des cellules photoélectriques 102, afin que l'opacimètre 30 soit pratiquement insensible au passage d'une matière en suspension ou aux remous du liquide à l'intérieur du tube 98. Ainsi, les signaux sortant de l'additionneur 104 et qui sont transmis au microprocesseur 34 au travers de l'interface 40 peuvent être utilisés par exemple pour déclencher l'une des alarmes 42 lorsqu'un seuil d'opacité préalablement affiché sur l'imprimante 36 est atteint.

Le microprocesseur 34 permet également, au travers de l'interface 40, lorsque l'appareil est en fonctionnement, de commander l'actionnement en continu de la pompe 28 et de l'opacimètre 30.

Lors de la mise sous tension de l'appareil qui vient d'être décrit en détail en se référant aux figures 1 à 3, le programme interne du microprocesseur 34 est conçu de préférence pour rappeler à l'opérateur, par exemple par l'intermédiaire d'un dispositif d'affichage prévu sur le clavier de commande 36, qu'il doit introduire un certain nombre de paramètres nécessaires au bon fonctionnement de l'appareil.

Ces paramètres sont notamment la date et l'heure, la périodicité des prélèvements conduisant à une prise d'échantillons et à une analyse au moyen des ensembles 22 et 24, la nature des analyses à effectuer par l'ensemble d'analyse 24, le seuil de chacune des alarmes 42, ainsi que les actions à réaliser lors de l'apparition de ces alarmes.

Lorsque tous les paramètres sont introduits, l'opacimètre 30 entre en action et fonctionne de façon continue jusqu'à l'arrêt de l'appareil. La pompe 28 est donc actionnée en continu et la surveillance de l'opacité des effluents liquides rejetés par la canalisation 10 est assurée de la manière décrite précédemment. Si un seuil d'opacité préalablement affiché est atteint, une alarme est déclenchée et le déclenchement de cette alarme peut s'accompagner d'une action sur les vannes 12 et 16 si cette action a été préalablement programmée.

Le contrôle en continu de l'opacité des effluents rejetés par la canalisation 10 s'accompagne, de façon intermittente, du prélèvement d'un échantillon par l'ensemble 22 et de l'analyse immédiate d'un échantillon prélevé simultanément par l'ensemble 24. Chacune de ces opérations de prélèvement s'accompagne d'une mesure de débit effectuée à l'aide du débitmètre 32 et d'une mesure du Ph assurée par le Ph-mètre 62. La périodicité de ces opérations est affichée initialement par l'opérateur et peut être, par exemple, d'environ 15 mn.

A chaque fois qu'un ordre de prélèvement est émis par le microprocesseur 34, la pompe 20 est actionnée pendant une durée suffisante pour permettre la purge des réseaux de prise d'échantillons des ensembles 22 et 24 et le remplissage des pipettes 44 et 64.

Plus précisément, dans un premier temps les vannes 46 et 54 sont ouvertes et la vanne 50 fermée, dans l'ensemble 22, de façon à purger la pipette 44.

Simultanément, les vannes 66, 72 et 96 sont ouvertes et les vannes 92, 78a, 78b, 78c sont fermées dans l'ensemble 24 afin de purger la pipette 64 et le tube d'analyse 68.

Lorsque les purges sont terminées, les vannes 54 et 72 sont fermées de façon à permettre le remplissage simultané des pipettes 44 et 64. A titre d'exemple, la contenance de la pipette 44 peut être d'environ 25 ml.

En même temps que deux échantillons des effluents liquides sont prélevés dans les pipettes 44 et 64, le débit instantané de ces effluents dans la canalisation 10 est mesuré par le débitmètre 32.

Lorsqu'un échantillon d'effluents se trouve dans la pipette 44, son Ph est mesuré par le Ph-mètre 62.

Les informations ainsi mesurées sur le débitmètre et sur le Ph-mètre sont transmises au microprocesseur 34 qui les mémorise afin de les afficher et de pouvoir les utiliser ultérieurement.

Avant que l'échantillon prélevé dans la pipette 44 ne soit introduit dans l'un des flacons vides 58, le moteur pas à pas 60 est actionné pour venir placer l'un de ces flacons sous le tube 48. L'échantillon est ensuite versé par gravité dans ce flacon par ouverture de la vanne 50 commandée par le microprocesseur 34.

Avant que l'échantillon prélevé dans la pipette 64 ne subisse les analyses préalablement affichées sur le clavier de commande 36, le tube d'analyse 68 est rincé par ouverture des vannes 92 et 96, au moyen du liquide de rinçage acheminé par le tube 90.

Lorsque le rinçage est terminé, la vanne 96 est fermée et une première analyse peut être effectuée sur l'échantillon prélevé dans la pipette 64. A cet effet, on verse par gravité une partie de cet échantillon dans le tube d'analyse 68, en ouvrant pendant un temps déterminé la vanne 72. Le réactif correspondant à l'analyse à effectuer est également versé par gravité à partir du réservoir 74a, 74b, 74c correspondant, en ouvrant pendant un temps déterminé la vanne 78a, 78b, 78c associée à ce réservoir. Simultanément, le moteur pas à pas 82 est actionné pour introduire dans le photomètre 80 le filtre 86a, 86b, 86c adapté à l'analyse à effectuer. Le photomètre 80 réalise alors la mesure de la concentration d'un élément chimique donné dans l'échantillon prélevé auparavant dans la pipette 64.

Lorsque cette analyse est terminée, le tube d'analyse 68 est à nouveau rincé de la manière décrite précédemment et une nouvelle fraction de l'échantillon 64 ainsi qu'une fraction d'un autre réactif sont injectées dans ce tube pour effectuer une autre analyse à l'aide du photomètre 80 préalablement équipé d'un autre filtre adapté à cette analyse.

Dans la pratique et à titre d'exemple, il est ainsi possible de mesurer la concentration d'éléments chimiques tels que le cadmium, le cuivre, le fer, le nickel, le chrome, le zinc, le cyanure et les fluorures sur un même échantillon prélevé à un moment donné dans la pipette 64.

Les informations fournies par le photomètre 80 sont transmises au fur et à mesure dans le microprocesseur 34, pour être mémorisées et affichées sur l'imprimante 38. A l'aide des concentrations et des débits mesurés, le microprocesseur calcule le poids de chacun des éléments chimiques surveillés contenu dans les effluents, sur une période déterminée. Pour chaque élément, il compare ce poids à un seuil préalablement affiché sur le clavier de commande 36 pour déclencher, si nécessaire, une alarme 42 correspondante et, le cas échéant, exercer une action préalablement programmée telle que la fermeture des vannes 12 et 16.

En réalisant des prélèvements à intervalles réguliers et relativement courts dans les effluents liquides rejetés par la canalisation 10 et en analysant immédiatement ces prélèvements dans l'ensemble 24, il est donc possible, à l'aide des mesures fournies par ailleurs par le débitmètre 32, de connaître avec une grande précision la quantité de certains éléments chimiques rejetée dans les effluents au cours d'une période donnée, par exemple, en une journée. Grâce à l'appareil qui vient d'être décrit, il est à noter que les résultats des analyses sont obtenus de façon très rapide et précise sans qu'il soit nécessaire d'avoir recours à un personnel qualifié.

De plus, lorsque les résultats des analyses effectuées de façon automatique par l'appareil sont anormaux ou soulèvent un doute quelconque, il est toujours possible de procéder ultérieurement à une analyse de l'échantillon correspondant contenu dans l'un des flacons 58 portés par le plateau 56. Etant donné que la date et l'heure de chaque prélèvement sont connues avec précision, on dispose ainsi d'un moyen de contrôle particulièrement fiable.

Par ailleurs, étant donné que les analyses sont effectuées isolément sur chacun des éléments chimiques à surveiller, la détection d'une anomalie concernant l'un de ces éléments peut permettre d'effectuer une intervention ponctuelle sur les seuls organes concernés par cet élément dans l'installation industrielle, sans arrêter pour autant l'ensemble de l'installation.

Enfin, il est à noter que l'appareil selon l'invention peut être monté sur toute installation industrielle rejetant des effluents liquides, quel que soit le niveau de compétance des utilisateurs.

Bien entendu, l'invention n'est pas limitée au mode de réalisation qui vient d'être décrit à titre d'exemple, mais en couvre toutes les variantes. Ainsi, le plateau tournant supportant les flacons destinés à recevoir les prélèvements peut être remplacé par un dispositif d'avance pas à pas de nature différente tel qu'un transporteur pas à pas. De même, on a vu que le photomètre peut être remplacé par un dispositif d'analyse équivalent tel qu'un spectromètre de masse.

## Revendications

1. Appareil pour l'analyse et la prise d'échantillons sur des effluents liquides sortant d'une installation industrielle par une canalisation de rejet (10), comprenant :
   – un premier tube de prélèvement (18) raccordé sur la canalisation de rejet, équipé d'un premier organe de pompage (20) et alimentant un ensemble de prise d'échantillons (22) ;
   – un moyen de mesure de débit (32) dans la canalisation de rejet ; et
   – un ensemble de commande (34) actionnant automatiquement le premier organe de pompage (20), l'ensemble de prise d'échantillons (22) et le moyen de mesure de débit (32) en fonction de paramètres et recevant des signaux délivrés par le moyen de mesure de débit (32) pour déterminer lesdites informations ;
caractérisé par le fait que, la canalisation de rejet (10) étant équipée d'une vanne (12) :
   – le premier tube de prélèvement (18) alimente simultanément un ensemble d'analyse (24) mesurant la concentration d'au moins un élément chimique dans les effluents, cet ensemble d'analyse comprenant une première pipette de prélèvement (64) interposée entre le premier tube de prélèvement (18) et un tube d'analyse (68), placé à un niveau inférieur à celui de la pipette, cette dernière comportant une vanne supérieure (66) et une vanne inférieure (72) actionnées par l'ensemble de commande, un tube de vidange (94), également équipé d'une vanne (96) actionnée par l'ensemble de commande, reliant le fond du tube d'analyse (68) à la canalisation de rejet (10), l'ensemble d'analyse (24) comprenant aussi des réservoirs (74a,74b,74c) contenant différents réactifs et situés à un niveau supérieur à celui du tube d'analyse (68), chaque réservoir étant relié au tube d'analyse par un tube (76a,76b,76c) équipé d'une vanne (78a,78b,78c) actionnée par l'ensemble de commande ;
   – un deuxième tube de prélèvement (26), raccordé sur la canalisation de rejet (10) et équipé d'un deuxième organe de pompage (28) alimente un opacimètre (30) ; et
   – l'ensemble de commande (34) est équipé d'un organe d'entrée de paramètres (36) et d'un organe de sortie d'informations (38), cet ensemble de commande actionnant aussi automatiquement le deuxième organe de pompage (28), l'ensemble d'analyse (24) et l'opacimètre (30), et recevant aussi des signaux délivrés par l'ensemble d'analyse (24) et par l'opacimètre (30).

2. Appareil selon la revendication 1, caractérisé par le fait qu'il comprend au moins une alarme (42) actionnée automatiquement par l'ensemble de commande (34) lorsque l'un au moins desdits signaux franchit un seuil constituant l'un desdits paramètres.

3. Appareil selon l'une quelconque des revendications 1 et 2, caractérisé par le fait que la vanne (12) équipant la canalisation de rejet (10) soit fermée automatiquement par l'ensemble de commande (34) lorsque l'un au moins desdits signaux franchit un seuil constituant l'un desdits paramètres.

4. Appareil selon l'une quelconque des revendications précédentes, caractérisé par le fait que l'ensemble de prise d'échantillon (22) comprend une deuxième pipette de prélèvement (44) reliée au premier tube de prélèvement (18) et placée de façon à se trouver en permanence au-dessus d'un flacon (58) porté par un dispositif d'avance pas à pas (56), la pipette comportant une vanne supérieure (46) et une vanne inférieure (50) actionnées par l'ensemble de commande (34), un tube de rinçage (52) également équipé d'une vanne (54) actionnée par l'ensemble de commande (34) reliant le fond de la pipette à la canalisation de rejet.

5. Appareil selon la revendication 4, caractérisé par le fait qu'un moyen de mesure de Ph (62), relié à l'ensemble de commande (34) est placé dans la deuxième pipette de prélèvenent (44).

6. Appareil selon l'une quelconque des revendications 4 et 5, caractérisé par le fait que le dispositif d'avance pas à pas comprend un plateau tournant (56) portant les flacons (58) et un moteur pas à pas (60) actionné par l'ensemble de commande (34) pour assurer une rotation pas à pas du plateau.

7. Appareil selon l'une quelconque des revendications précédentes, caractérisé par le fait qu'un autre tube (90) équipé d'une vanne (92) actionnée par l'ensemble de commande relie le tube d'analye (68) à une source de liquide de rinçage.

8. Appareil selon l'une quelconque des revendications précédentes, caractérisé par le fait que l'ensemble d'analyse (24) comprend un dispositif d'analyse (80) mesurant la concentration dudit élément chimique à l'intérieur du tube d'analyse (68).

9. Appareil selon la revendication 8, caractérisé par le fait que le dispositif d'analyse comprend un photomètre (80) muni d'une source lumineuse (88) et d'un sélecteur de filtres (84) entraîné par un moteur pas à pas (82) actionné par l'ensemble de commande.

10. Appareil selon l'une quelconque des revendications précédentes, caractérisé par le fait que l'opacimètre (30) comprend un tube transparent (98) dans lequel circulent les effluents, au moins une source lumineuse (100) placée latéralement le long du tube d'analyse, des cellules photoélectriques (102) placées en vis-à-vis de la source lumineuse, de l'autre côté du tube d'analyse, et un moyen (104) pour calculer la somme des signaux délivrés par les cellules, ce moyen étant actionné par l'ensemble de commande, qui reçoit en retour un signal représentatif de cette somme.

## Claims

1. Apparatus for the analysis and taking of samples of liquid effluents leaving an industrial plant via a waste pipeline (10), comprising:
– a first sampling pipe (18) connected to the waste pipeline, equipped with a first piping member (20) and feeding a unit for taking samples (22);
– a means for measuring flow rate (12) in the waste pipeline; and
– a control unit (14) automatically actuating the first pumping member (20), the unit for taking samples (22) and the means for measuring flow rate (32) as a function of parameters and receiving signals supplied by the means for measuring flow rate (32) in order to determine the said data; characterised in that, the waste pipeline (10) being equipped with a valve (12):
– the first sampling pipe (18) simultaneously feeds an analysis unit (24) measuring the concentration of at least one chemical element in the effluents, this analysis unit comprising a first sampling pipette (64) placed between the first sampling pipe (18) and an analysis pipe (68) placed at a level lower than that of the pipette, the latter comprising an upper valve (66) and a lower valve (72) which are actuated by the control unit, a draining pipe (94), also equipped with a valve (96) actuated by the control unit, connecting the bottom of the analysis pipe (68) to the waste pipeline (10), the analysis unit (24) also comprising storage vessels (74a,74b,74c) containing various reactants and situated at a level higher than that of the analysis pipe (68), each storage vessel being connected to the analysis pipe by a pipe (76a,76b,76c) equipped with a valve (78a,78b,78c) actuated by the control unit;
– a second sampling pipe (26) connected to the waste pipeline (10) and equipped with a second pumping member (28) feeds a turbidimeter (30); and
– the control unit (34) is equipped with a parameter input member (36) and a data output member (38), this control unit also automatically actuating the second pumping member (28), the analysis unit (24) and the turbidimeter (30) and also receiving signals supplied by the analysis unit (24) and by the turbidimeter (30).

2. Apparatus according to Claim 1, characterised in that it comprises at least one alarm (42) automatically actuated by the control unit (34) when at least one of the said signals crosses a threshold constituting one of the said parameters.

3. Apparatus according to either of Claims 1 and 2, characterised in that the valve (12) fitted to the waste pipeline (10) is automatically closed by the control unit (34) when at least one of the said signals crosses a threshold constituting one of the said parameters.

4. Apparatus according to any one of the preceding claims, characterised in that the unit for taking a sample (22) comprises a second sampling pipette (44) connected to the first sampling pipe (18) and placed so that it is continuously above a flask (58) carried by a device for stepwise forward travel (56), the pipette comprising an upper valve (46) and a lower valve (50) which are actuated by the control unit (34), a rinsing pipe (52) also equipped with a valve (54) actuated by the control unit (34) connecting the bottom of the pipette to the waste pipeline.

5. Apparatus according to Claim 4, characterised in that a means for measuring pH (62) connected to the control unit (34) is placed in the second sampling pipette (44).

6. Apparatus according to either of Claims 4 and 5, characterised in that the device for stepwise forward travel comprises a rotating tray (56) carrying flasks (58) and a stepping motor (60) actuated by the control unit (34) in order to ensure a stepwise rotation of the tray.

7. Apparatus according to any one of the preceding claims, characterised in that another pipe (90) equipped with a valve (92) actuated by the control unit connects the analysis pipe (68) to a source of rinsing liquid.

8. Apparatus according to any one of the preceding claims, characterised in that the analysis unit (24) comprises an analysis device (80) measuring the concentration of the said chemical element inside the analysis pipe (68).

9. Apparatus according to Claim 8, characterised in that the analysis device comprises a photometer (80) provided with a source of light (88) and with a filter selector (84) driven by a stepping motor (82) actuated by the control unit.

10. Apparatus according to any one of the preceding claims, characterised in that the turbidimeter (30) comprises a transparent pipe (98) in which the effluents flow, at least one source of light (100) placed laterally along the analysis pipe, photoelectric cells (102) placed opposite the source of light, on the other side of the analysis pipe, and a means (104) for calculating the sum of the signals supplied by the cells, this means being actuated by the control unit, which in return receives a signal which is representative of this sum.

**Patentansprüche**

1. Gerät zur Analyse und zur Probenentnahme bei flüssigen Abwässern, welche durch eine Abflußleitung (10) aus einer industriellen Anlage kommen, umfassend:
   – ein erstes, an die Abflußleitung angeschlossenes Proberohr (18), welches mit einer ersten Pumpeinrichtung (20) ausgestattet ist und eine Probenentnahmeeinheit (22) speist,
   – eine Meßeinrichtung für die Ausströmungsmenge (32) in der Abflußleitung, und
   – eine Steuereinheit (34), welche automatisch die erste Pumpeinrichtung (20), die Probenentnahmeeinheit (22) und die Meßvorrichtung für die Ausströmungsmenge (32) in Abhängigkeit von Parametern betätigt und die von der Meßeinrichtung ausgesandten Signale für die Ausströmungsmenge (32) empfängt, um die genannten Informationen zu bestimmen;
   gekennzeichnet durch die Tatsache, daß die Abflußleitung (10) mit einem Schieber (12) ausgestattet ist:
   – das erste Proberohr (18) speist gleichzeitig eine Analyseneinheit (24), welche die Konzentration mindestens eines chemischen Element in den Abwässern mißt, wobei diese Analyseneinheit (24) eine erste Probepipette (64) umfaßt, die zwischen dem ersten Proberohr (18) und einem Analyserohr (68) eingeschoben ist, welches auf einem niedrigeren Niveau als dem der Pipette angebracht ist, wobei letztere einen oberenn Schieber (66) und einen unteren Schieber (72), welche von der Steuereinheit betätigt werden, ein ebenfalls mit einem Schieber (96) ausgestattetes und von der Steuereinheit betätigtes Auslaufrohr (94) umfaßt, welches den Boden des Analyserohres (68) mit der Abflußleitung (10) verbindet, wobei die Analyseneinheit (24) auch Analysenbehälter (74a, 74b, 74c) umfaßt, welche verschiedene Reagenzien enthalten und sich auf einem höheren Niveau befinden als das des Analyserohrs (68), und jedes Reservoir mit dem Analyserohr durch jeweils ein Rohr (76a, 76b, 76c), das mit einem von der Steuereinheit betätigten Schieber (78a, 78b, 78c) ausgestattet ist, verbunden ist;
   – ein zweites Proberohr (26), welches an die Abflußleitung (10) angeschlossen und mit einer zweiten Pumpeinrichtung (28) ausgestattet ist, speist einen Trübungsmesser (30); und
   – die Steuereinheit (34) ist mit einer Einrichtung für den Parametereingang (36) und mit einer Einrichtung für den Informationsausgang (38) ausgestattet, wobei diese Steuereinheit auch automatisch die zweite Pumpeinrichtung (28), die Analyseneinheit (24) und den Trübungsmesser (30) betätigt und auch Signale, welche von der Analyseneinheit (24) und vom Trübungsmesser (30) ausgesandt werden, empfängt.

2. Gerät nach Anspruch 1, gekennzeichnet durch die Tatsache, daß es mindestens einen Alarm (42) umfaßt, welcher automatisch von der Steuereinheit (34) betätigt wird, wenn mindestens eines der genannten Signale eine Schwelle, die einen der genannten Parameter darstellt, überschreitet.

3. Gerät nach einem der Ansprüche 1 und 2, gekennzeichnet durch die Tatsache, daß der Schieber (12), mit welchem die Abflußleitung (10) ausgestattet ist, automatisch von der Steuereinheit (34) geschlossen wird, wenn mindestens eines der genannten Signale eine Schwelle, die einen der genannten Parameter darstellt, überschreitet.

4. Gerät nach einem der vorangegangenen Ansprüche, gekennzeichnet durch die Tatsache, daß die Probenentnahmeeinheit (22) eine zweite Probepipette (44) umfaßt, welche mit dem ersten Proberohr (18) verbunden ist und so angebracht ist, daß sie sich ständig unter einem von einer Vorrichtung zur schrittweisen Verstellung (56) getragenen Kolben (58) befindet, wobei die Pipette einen oberen Schieber (46) und einen unteren Schieber (50), welcher von der Steuereinheit (34) betätigt werden, ein ebenfalls mit einem Schieber (54), der von der Steuereinheit (34) betätigt wird, ausgestattetes Spülrohr (52) umfaßt, welches den Boden der Pipette mit der Abflußleitung verbindet.

5. Gerät nach Anspruch 4, gekennzeichnet durch die Tatsache, daß eine an die Steuereinheit (34) angeschlossene Meßvorrichtung für den pH (62) in der zweiten Probepipette (44) angebracht ist.

6. Gerät nach einem der Ansprüche 4 und 5, gekennzeichnet durch die Tatsache, daß die Vorrichtung zur schrittweisen Verstellung einen Drehteller (56) umfaßt, welcher die Kolben (58) und einen von der Steuereinheit (34) betätigten Schrittmotor (60) trägt, um eine schrittweise Rotation des Tellers zu gewährleisten.

7. Gerät nach einem der vorangegangenen Ansprüche, gekennzeichnet durch die Tatsache, daß ein weiteres Rohr (90), welches mit einem von der Steuereinheit betätigten Schieber (92) ausgestattet ist, das Analyserohr (68) mit einer Spülflüssigkeitsquelle verbindet.

8. Gerät nach einem der vorangegangenen Ansprüche, gekennzeichnet durch die Tatsache, daß die Analyseneinheit (24) eine Analyseneinrichtung (80) umfaßt, welche die Konzentration des genannten chemischen Elements im Inneren des Analyserohres (68) mißt.

9. Gerät nach Anspruch 8, gekennzeichnet durch die Tatsache, daß die Analyseneinrichtung ein Photometer (80), welches mit einer Lichtquelle (88) und mit einem Filterwähler (84) ausgestattet ist, welcher von einem von der Steuereinheit (34) betätigten Schrittmotor (82) angetrieben wird, umfaßt.

10. Gerät nach einem der vorangegangenen Ansprüche, gekennzeichnet durch die Tatsache, daß der Trübungsmesser (30) ein transparentes Rohr (98), in welchem die Abwässer fließen, mindestens eine seitlich längs des Analyserohres angebrachte Lichtquelle (100), gegenüber der Lichtquelle auf der anderen Seite des Analyserohres angebrachte photoelektrische Zellen (102) und eine Vorrichtung (104) zur Berechnung der Summe der von den Zellen abgegebenen Signale umfaßt, wobei diese Vorrichtung von der Steuereinheit (34) betätigt wird, welche wiederum ein für diese Summe charakteristisches Signal empfängt.

FIG. 1

# FIG. 2

FIG. 3